# EUROPEAN PATENT APPLICATION

(11) **EP 4 691 983 A1**
(43) Date of publication of application: **11.02.2026**
(21) Application number: 25191456.0
(22) Date of filing: 24.07.2025
(51) Int. Cl.: C01B 35/10, C02F 1/58, B01D 3/00, B01D 5/00

(54) **METHOD AND APPARATUS FOR PREPARING LOW-FLUORINE BORON-10 ENRICHED BORIC ACID**

(30) Priority: 30.07.2024 CN 202411034836
(71) Applicant: Shandong Heyi Gas Co., Ltd., Dongying City, Shandong Province 257500 (CN)
(72) Inventor: ZHANG, Shujiang, Dongying City, Shandong Province, 257500 (CN); YU, Chunyu, Dongying City, Shandong Province, 257500 (CN); LI, Kangning, Dongying City, Shandong Province, 257500 (CN); YANG, Jun, Dongying City, Shandong Province, 257500 (CN); XU, Baoyun, Dongying City, Shandong Province, 257500 (CN); WANG, Zhejun, Dongying City, Shandong Province, 257500 (CN); YAN, Xiuxiang, Dongying City, Shandong Province, 257500 (CN)
(74) Representative: Schneiders & Behrendt Bochum

(57) **Abstract**

Provided are a method and an apparatus for preparing a low-fluorine boron-10 enriched boric acid. The method includes subjecting a raw material liquid to a reflux treatment, subjecting a gaseous material produced during the reflux treatment to a defluorination treatment by using a defluorination agent, subjecting a resulting defluorinated material to rectification and then condensation, and returning a resulting condensed material to a cycle and conducting the reflux treatment, where the raw material liquid includes a high-fluorine boron-10 enriched boric acid and methanol; after the reflux treatment is completed, maintaining distillation and receiving a resulting distillate fraction, and mixing the resulting distillate fraction with water and subjecting a resulting mixture to a hydrolysis treatment to obtain a hydrolysate; and concentrating the hydrolysate, and subjecting a resulting concentrated solution to solid-liquid separation to obtain the low-fluorine boron-10 enriched boric acid.

## Description

### TECHNICAL FIELD

The present disclosure relates to the technical field of boric acid preparation, and in particular to a method and an apparatus for preparing a low-fluorine boron-10 enriched boric acid.

### BACKGROUND

In nature, boron has two stable isotopes, boron-10 and boron-11. Boron-10 exhibits an abundance of 19.8% and exhibits a very strong thermal neutron absorption capacity. Consequently, boron-10 finds extensive applications in fields of nuclear power, medicine, military equipment, and electronics. For example, in the field of nuclear power, the addition of boron-10 enriched boric acid (the abundance of boron -10 is usually required to be 60-90%) to a nuclear power primary circuit reduces boric acid consumption, mitigates equipment corrosion, and decreases wastewater generation.

Boron-10 enriched boric acid typically undergoes isotope separation with boron trifluoride, resulting in boron-10 boric acid enriched products containing fluoride ions. While the nuclear power primary circuit is a heavy water reactor. When the boron -10 enriched boric acid with a high fluoride ion content is used in the nuclear power primary circuit, it will pose a threat to the safety of the nuclear power primary circuit. Therefore, the boron -10 enriched boric acid used in the nuclear power primary circuit requires fluoride ion content ≤ 0.4 ppm.

In the preparation of boron-10 enriched boric acid for nuclear power primary circuits in the prior art, in order to reduce the fluoride ion content, a secondary treatment such as recrystallization is usually required, the product yield is low and the operation is complicated.

### SUMMARY

The object of the present disclosure is to provide a method and an apparatus for preparing a low-fluorine boron-10 enriched boric acid. The low-fluorine boron-10 enriched boric acid prepared by the method of the present disclosure exhibits a high yield and a simple operation.

In order to achieve the above object, the present disclosure provides the following technical solutions:
The present disclosure provides a method for preparing a low-fluorine boron-10 enriched boric acid, including the following steps:
subjecting a raw material liquid to a reflux treatment, subjecting a gaseous material produced during the reflux treatment to a defluorination treatment by using a defluorination agent, then subjecting a resulting defluorinated material to rectification and then condensation, and returning a resulting condensed material to a cycle and conducting the reflux treatment, where the raw material liquid comprises a high-fluorine boron-10 enriched boric acid and methanol, the high-fluorine boron-10 enriched boric acid having a fluoride ion content of ≥ 200 ppm;
after the reflux treatment is completed, maintaining distillation and receiving a resulting distillate fraction, and mixing the resulting distillate fraction with water and subjecting a resulting mixture to a hydrolysis treatment to obtain a hydrolysate; and
concentrating the hydrolysate, and subjecting a resulting concentrated solution to solid-liquid separation to obtain the low-fluorine boron-10 enriched boric acid.

In some embodiments, a molar ratio of the high-fluorine boron-10 enriched boric acid to the methanol is in a range of 1 : 10 to 1 : 23.

In some embodiments, the reflux treatment is conducted at a heating temperature of 55°C to 60°C and a condensation temperature of -8°C to -15°C; the reflux treatment is conducted for 4 to 6 h; and the reflux treatment is conducted under stirring at a speed of 50rpm to 100 rpm.

In some embodiments, the defluorination agent comprises an ion exchange resin or aluminum sulfate.

In some embodiments, a mass ratio of the low-fluorine boron-10 enriched boric acid to the water is in a range of 3 : 100 to 5 : 100, based on a mass of the low-fluorine boron-10 enriched boric acid; the water is at a temperature of 40°C to 50°C.

In some embodiments, the hydrolysis treatment is conducted at a temperature of 40°C to 50°C; and the hydrolysis treatment is conducted under stirring at a speed of 50rpm to 100 rpm.

In some embodiments, the concentration is conducted by reduced pressure concentration; and the concentration is conducted under stirring at a speed of 50 rpm to 100 rpm.

In some embodiments, the method further includes: after the solid-liquid separation, drying a wet substance obtained by the solid-liquid separation to obtain the low-fluorine boron-10 enriched boric acid.

The present disclosure provides an apparatus for preparing a low-fluorine boron-10 enriched boric acid, including a reflux unit, a hydrolysis tank, and a solid-liquid separation unit connected in sequence, where
the reflux unit includes a reaction tank, a packed rectification column, and a condenser connected in sequence, where the packed rectification column includes, an adsorption section and a rectification section in sequence, the adsorption section being packed with a defluorination agent and being in communication with the reaction tank; and a liquid outlet of the condenser is in communication with a reflux inlet of the rectification section and a distillate inlet of the hydrolysis tank, respectively.

In some embodiments, the apparatus further includes a water storage tank, a methanol storage tank, and a drying unit, where
a water outlet of the water storage tank is in communication with a water inlet of the hydrolysis tank, and a water inlet of the water storage tank is in communication with a water outlet of the hydrolysis tank;
a methanol inlet of the methanol storage tank is in communication with a methanol outlet of the hydrolysis tank; and
a feed inlet of the drying unit is in communication with a solid discharge outlet of the solid-liquid separation unit.

The present disclosure provides a method for preparing a low-fluorine boron-10 enriched boric acid, including the following steps: subjecting a raw material liquid to a reflux treatment, subjecting a gaseous material produced during the reflux treatment to a defluorination treatment by using a defluorination agent, then subjecting a resulting defluorinated material to rectification and then condensation, and returning a resulting condensed material to a cycle and conducting the reflux treatment, where the raw material liquid includes a high-fluorine boron-10 enriched boric acid and methanol, the high-fluorine boron-10 enriched boric acid having a fluoride ion content of ≥ 200 ppm; after the reflux treatment is completed, maintaining distillation and receiving a resulting distillate fraction, and mixing the resulting distillate fraction with water and subjecting a resulting mixture to hydrolysis to obtain a hydrolysate; and concentrating the hydrolysate and subjecting a resulting concentrated solution to solid-liquid separation to obtain the low-fluorine boron-10 enriched boric acid. According to the present disclosure, a high-fluorine boron-10 enriched boric acid reacts with methanol to generate trimethyl borate vapor (specifically in the form of trimethyl borate-methanol azeotrope). Fluoride ions in the gas phase are easier to separate. Fluoride ions in the gas phase can be effectively adsorbed and removed by using a defluorination agent without secondary treatment such as recrystallization, and finally, a low-fluorine boron -10 enriched boric acid is obtained in high yield. The results of the examples show that the low-fluorine boron-10 enriched boric acid prepared by the method of the present disclosure exhibits a product yield of 95.73% to 98.60% and a fluoride ion content of 0.21 ppm to 0.31 ppm.

### BRIEF DESCRIPTION OF THE DRAWINGS

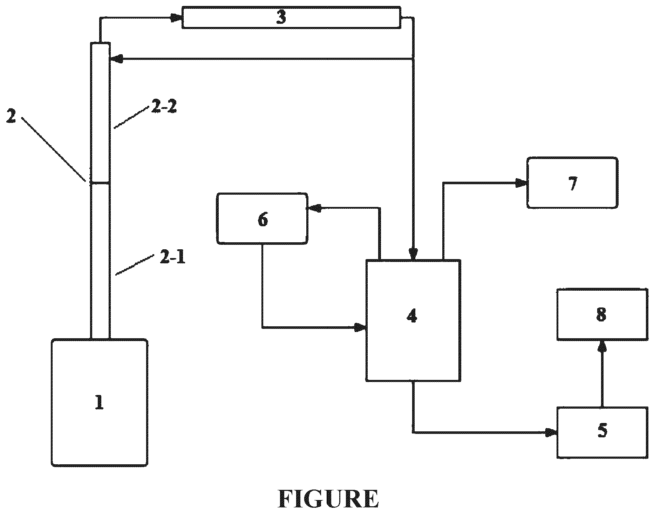

FIGURE shows a schematic structural diagram of the apparatus for preparing a low-fluorine boron-10 enriched boric acid according to an embodiment of the present disclosure. In the figure, 1 denotes a reaction tank, 2 denotes a packed rectification column, 2-1 denotes an adsorption section, 2-2 denotes a rectification section, 3 denotes a condenser, 4 denotes a hydrolysis tank, 5 denotes a solid-liquid separation unit, 6 denotes a water storage tank, 7 denotes a methanol storage tank, and 8 denotes a drying unit.

### DETAILED DESCRIPTION OF THE EMBODIMENTS

The present disclosure provides a method for preparing a low-fluorine boron-10 enriched boric acid, the method including the following steps:
subjecting a raw material liquid to a reflux treatment, subjecting a gaseous material produced during the reflux treatment to a defluorination treatment by using a defluorination agent, and then subjecting a resulting defluorinated material to rectifying and the condensing, returning a resulting condensed material to a cycle and conducting the reflux treatment, where the raw material liquid comprises a high-fluorine boron-10 enriched boric acid and methanol, the high-fluorine boron-10 enriched boric acid having a fluoride ion content of ≥ 200 ppm;
after the reflux treatment is completed, maintaining distillation and receiving a resulting distillate fraction, and mixing the resulting distillate fraction with water and subjecting a resulting mixture to hydrolysis to obtain a hydrolysate; and
concentrating the hydrolysate, and then subjecting a resulting concentrated solution to solid-liquid separation to obtain the low-fluorine boron-10 enriched boric acid.

In the present disclosure, unless otherwise specified, all raw materials and reagents are commercially available and well known to those skilled in the art.

According to the present disclosure, high-fluorine boron-10 enriched boronic acid reacts with methanol to produce a trimethyl borate vapor (specifically in the form of a trimethyl borate-methanol azeotrope). A reaction equation is: H₃BO₃+3CH₃OH=B(OCH₃)₃+3H₂O. Fluoride ions in the gas phase are easier to separate. Fluoride ions in the gas phase can be effectively adsorbed and removed by using a defluorination agent, and finally, a low-fluorine boron-10 enriched boric acid is obtained. The preparation of a low-fluorine boron-10 enriched boric acid without change or production of harmful substances by the method of the present disclosure exhibits a simple operation, high product yield and purity, and a low fluoride ion content, suitable for use in fields of nuclear industry, nuclear medicine, semiconductor manufacturing, etc., such as in the nuclear power primary circuit, and exhibits enormous economic benefits. The low-fluorine boron-10 enriched boric acid of the present disclosure specifically refers to a fluoride ion content of ≤ 0.4 ppm, preferably ≤ 0.31 ppm, more preferably ≤ 0.29 ppm, and further preferably ≤ 0.21 ppm. The method of the present disclosure is described in detail below.

In the present disclosure, the method includes subjecting a raw material liquid to a reflux treatment, subjecting a gaseous material produced during the reflux treatment to a defluorination treatment by using a defluorination agent, then subjecting a resulting defluorinated material to rectification and then condensation, and returning a resulting condensed material to a cycle and conducting the reflux treatment, where the raw material liquid comprises a high-fluorine boron-10 enriched boric acid and methanol. In some embodiments, the high-fluorine boron-10 enriched boric acid has a fluoride ion content of ≥ 200 ppm, preferably from 200 ppm to 210 ppm, more preferably 202 ppm to 205 ppm. In some embodiments, the abundance of boron-10 in the high-fluorine boron-10 enriched boric acid is 60% to 90%, more preferably from 60% to 80%, more preferably 60% to 70%. In an embodiment of the present disclosure, the high-fluorine boron-10 enriched boric acid has a fluoride ion content of 202.55 ppm, a boron-10 abundance of 60%, a boric acid content of 100.23%, and a water insoluble content of 10 ppm. In some embodiments of the present disclosure, a molar ratio of the high-fluorine boron-10 enriched boric acid to the methanol is in a range of 1 : 10 to 1 : 23, specifically 1 : 11, 1 : 12, 1 : 13, 1 : 14, 1 : 15, 1 : 16, 1 : 17, 1 : 18, 1 : 19, 1 : 20, 1 : 21, 1 : 22 or 1 : 23. In some embodiments of the present disclosure, the reflux treatment is conducted at a heating temperature of 55°C to 60°C, more preferably 55°C to 58°C and a condensation temperature of -8°C to -15°C, more preferably -12°C to -15°C; the reflux treatment is conducted for 4 to 6 h, specifically 4 h, 4.5 h, 5 h, 5.5 h or 6 h. In some embodiments, the reflux treatment is conducted in a packed rectification column, with a reflux treatment time starting when an overhead temperature of the packed rectification column reaches 51°C to 55°C. In some embodiments, the reflux treatment is conducted under stirring at a speed of preferably 50 rpm to 100 rpm, more preferably 50 rpm to 70 rpm. No fraction is received during the reflux treatment described in the present disclosure. In some embodiments of the present disclosure, the defluorination agent comprises an ion exchange resin or aluminum sulfate. In some embodiments, the ion exchange resin is Tulsimer^{®} CH-87 ion exchange resin or TA 466D ion exchange resin. In an embodiment of the present disclosure, the Tulsimer^{®} CH-87 ion exchange resin and TA 466D ion exchange resin are both purchased from COHESION (Beijing) Technology Co., Ltd. The defluorination agent used in some embodiments of the present disclosure exhibits a strong adsorption capacity for fluoride ions and can also adsorb other impurities (e.g. chloride ions, nitrate ions, sulfate ions, phosphate ions, silver ions, iron ions, nickel ions, calcium ions, potassium ions, sodium ions, magnesium ions, etc.), ensuring that a high purity product is obtained; furthermore, the defluorination agent used in some embodiments of the present disclosure is relatively safe and environmentally friendly, and no other harmful substances are produced during use; in addition, the defluorination agent used in some embodiments of the present disclosure is relatively inexpensive, convenient to use, and exhibits high economic benefits. In some embodiments of the present disclosure, a mass of the defluorination agent is greater than a mass of a fluoride in the highly fluorine rich boron-10 boric acid.

In the present disclosure, after the reflux treatment is completed, a distillation is maintained and a resulting distillate fraction is received, and the resulting distillate fraction is mixed with water and a resulting mixture is subjected to hydrolysis treatment to obtain a hydrolysate. In some embodiments of the present disclosure, the water is ultrapure water; a mass ratio of the low-fluorine boron-10 enriched boric acid to the water is in a range of 3 : 100 to 5 : 100, more preferably 4 : 100, based on a mass of the low -fluorine boron-10 enriched boric acid; and the water is at a temperature of 40°C to 50°C, more preferably 45°C to 50°C. In the present disclosure, after the reflux treatment is completed, the distillation is maintained and the resulting distillate fraction is received. In some embodiments of the present disclosure, the resulting distillate fraction is directly received into water previously heated to 40°C to 50°C for the hydrolysis treatment. In the present disclosure, trimethyl borate and methanol would form a trimethyl borate-methanol azeotrope with a boiling point of 54°C, the azeotropic ratio being a mass ratio of trimethyl borate to methanol of 7 : 3. A receiving amount of the resulting distillate fraction in some embodiments of the present disclosure is specifically an amount of trimethyl borate - methanol azeotrope formed by trimethyl borate and methanol. In some embodiments of the present disclosure, the hydrolysis treatment is conducted at a temperature of 40°C to 50°C, more preferably, 45°C to 50°C; the hydrolysis treatment is regarded as completed upon the receipt of the resulting distillate fraction; and the hydrolysis treatment is conducted under stirring, preferably at a speed of 50 rpm to 100 rpm, more preferably 50 rpm to 70 rpm.

After obtaining the hydrolysate, in the present disclosure, the hydrolysate is concentrated and then a resulting concentrated solution is subjected to solid-liquid separation to obtain the low-fluorine boron-10 enriched boric acid. In some embodiments of the present disclosure, the concentration is conducted by reduced pressure concentration; and the concentration is conducted under stirring, preferably at a speed of 50 rpm to 100 rpm, more preferably 50 rpm to 70 rpm. In some embodiments of the present disclosure, excess methanol and water are distilled out by reduced pressure concentration (specifically, methanol is roughly distilled at the boiling point of methanol first, then water is roughly distilled at the boiling point of water, followed by further separation of the distilled methanol-water mixture to obtain methanol and water, respectively), where during distillation under reduced pressure, crystals continue to precipitate, and the low -fluorine boron-10 enriched boric acid is obtained by solid-liquid separation. In some embodiments of the present disclosure, the solid-liquid separation is conducted by filtration or centrifugation. In some embodiments of the present disclosure, the method further comprises: after the solid-liquid separation, drying a wet substance obtained by the solid-liquid separation to obtain the low-fluorine boron-10 enriched boric acid. In some embodiments of the present disclosure, the drying is conducted by vacuum drying, and the drying is conducted at a temperature of 60°C to 70°C, more preferably 65°C; and the drying is conducted for 4 h to 6 h, more preferably 5 h.

The present disclosure provides an apparatus for preparing a low-fluorine boron-10 enriched boric acid, including a reflux unit, a hydrolysis tank, and a solid-liquid separation unit connected in sequence, where the reflux unit includes a reaction tank, a packed rectification column, and a condenser connected in sequence, where the packed rectification column includes an adsorption section and a rectification section in sequence, the adsorption section being packed with a defluorination agent and being in communication with the reaction tank; and a liquid outlet of the condenser is in communication with a reflux inlet of the rectification section and a distillate inlet of the hydrolysis tank, respectively.

As an embodiment of the present disclosure, the reaction tank includes a methanol feed inlet and a boric acid feed inlet disposed in a sidewall and a material channel disposed at the top, where the material channel of the reaction tank is in communication with the adsorption section of the packed rectification column for refluxing treatment. In some embodiments of the present disclosure, the adsorption section is packed with a defluorination agent. In the present disclosure, there are no particular limitations on the manner of packing with the defluorination agent, as long as the defluorination agent can be fixed and fully in contact with gaseous materials generated in the reaction tank to realize defluorination treatment. As an embodiment of the present disclosure, the rectification section is provided with a wire mesh packing inside. In the present disclosure, there are no particular limitations on the specification of the wire mesh packing, a wire mesh packing with a specification well known to those skilled in the art may be used. As an embodiment of the present disclosure, the adsorption section in the packed rectification column is provided with material channels through the bottom and the top, in communication with the reaction tank and the rectification section, respectively, for refluxing treatment. As an embodiment of the present disclosure, the rectification section of the packed rectification column is provided with material channels through the bottom and the top, in communication with the adsorption section and the condenser, respectively, for refluxing treatment. In some embodiments of the present disclosure, a liquid outlet of the condenser is in communication with a reflux inlet of the rectification section and a distillate inlet of the hydrolysis tank, respectively, in particular, the liquid outlet of the condenser is in communication with only the reflux inlet of the rectification section during the reflux treatment, i.e, refluxing the distillate fraction to the packed rectification column without receiving the distillate fraction in the hydrolysis tank; and after the reflux treatment is completed, the liquid outlet of the condenser is only in communication with the distillate inlet of the hydrolysis tank, i.e, not returning the distillate fraction to the packed rectification column, and receiving the distillate fraction in the hydrolysis tank and conducting hydrolysis treatment and concentration.

As an embodiment of the present disclosure, the solid-liquid separation unit is a filtration unit or a centrifuge unit, enabling solid-liquid separation.

As an embodiment of the present disclosure, the apparatus further includes a water storage tank and a methanol storage tank for recovering the water and the methanol produced during concentration, where a water outlet of the water storage tank is in communication with a water inlet of the hydrolysis tank, and a water inlet of the water storage tank is in communication with a water outlet of the hydrolysis tank, i.e., the water produced during concentration can be reused for hydrolysis treatment; and a methanol inlet of the methanol storage tank is in communication with a methanol outlet of the hydrolysis tank.

As an embodiment of the present disclosure, the apparatus further includes a drying unit. A feed inlet of the drying unit is in communication with a solid discharge outlet of the solid-liquid separation unit, and is configured to dry wet substances obtained after solid-liquid separation.

FIGURE shows a schematic structural diagram of the apparatus for preparing a low-fluorine boron-10 enriched boric acid according to an embodiment of the present disclosure. In the figure, 1 denotes a reaction tank, 2 denotes a packed rectification column, 2-1 denotes an adsorption section, 2-2 denotes a rectification section, 3 denotes a condenser, 4 denotes a hydrolysis tank, 5 denotes a solid-liquid separation unit, 6 denotes a water storage tank, 7 denotes a methanol storage tank, and 8 denotes a drying unit. The technical solutions in the present disclosure will be described clearly and completely below with reference to the examples of the present disclosure. Apparently, the described examples are merely some, rather than all of the examples of the present disclosure. On the basis of the embodiments of the present disclosure, all other embodiments that can be obtained by those of ordinary skill in the art without creative efforts shall fall within the scope of the present disclosure.

The high-fluorine boron-10 enriched boric acids used in the following Examples and Comparative Examples were the same batch, with a fluoride ion content of 202.55 ppm, a boron-10 abundance of 60%, a boric acid content of 100.23%, and a water insoluble content of 10 ppm.

### Example 1

123.6 g of the high -fluorine boron-10 enriched boric acid was weighed and charged to a reaction tank, and 1280 g of methanol was fed into the reaction tank at a molar ratio of boric acid to methanol of 1 : 20. The stirring was initiated, with a stirring speed maintained at 50 rpm, the boric acid was reacted with methanol at a reaction temperature of 55°C to form a trimethyl borate-methanol azeotrope, the trimethyl borate-methanol azeotrope was successively passed through an adsorption section of a packed rectification column (internally packed with an ion exchange resin, specifically Tulsimer^{®} CH-87 ion exchange resin, purchased from COHESION (Beijing, China) Technology Co., Ltd.. A mass of the ion exchange resin being greater than a mass of a fluoride in the high fluorine boron-10 enriched boric acid) and a rectification section (with a wire mesh packing disposed inside), and then passed through a condenser and refluxed to the reaction tank, where condensation was conducted at a temperature of -15°C, and reflux was conducted for 4 h, starting when an overhead temperature of the packed rectification column reached 55°C.

3090 g of ultrapure water was previously added to a hydrolysis tank, in which a temperature was set at 50°C, and a stirring speed was set at 50 rpm. After the reflux was completed, a distillate fraction was received in the hydrolysis tank, and after receiving 300 g of the distillate fraction, receiving was stopped. Materials in the hydrolysis tank were subjected to distillation under reduced pressure to distill off excess methanol and water (specifically, methanol was roughly distilled at the boiling point of methanol first, then water was roughly distilled at the boiling point of water, followed by further separation of distilled methanol-water mixture, and obtained methanol and water were respectively stored in a methanol storage tank and a water storage tank), where during distillation under reduced pressure, crystals continued to precipitate. After the distillation under reduced pressure was completed, a resulting solid-liquid mixture in the hydrolysis tank was filtered by a filtration unit, and a resulting filter cake was dried in a drying unit under vacuum at 65°C for 5 h to obtain 121.13 g of product (i.e., low-fluorine boron-10 enriched boric acid), with a yield of 98.00%.

### Example 2

123.6 g of the high-fluorine boron-10 enriched boric acid was weighed into a reaction tank, 1152 g of methanol was fed into the reaction tank at a molar ratio of boric acid to methanol of 1 : 18. The stirring was initiated, with a stirring speed maintained at 50 rpm, the boric acid was reacted with methanol at a reaction temperature of 55°C to form a trimethyl borate-methanol azeotrope, the trimethyl borate-methanol azeotrope was successively passed through an adsorption section of a packed rectification column (internally packed with aluminum sulfate, which had a mass greater than that of a fluoride in the high-fluorine boron-10 enriched boric acid) and a rectification section (provided internally with a wire mesh packing), and then through a condenser and refluxed to the reaction tank, where condensation was conducted at a temperature of -15°C, and reflux was conducted for 4 h, starting when an overhead temperature of the packed rectification column reached 55°C.

3090 g of ultrapure water was previously added to a hydrolysis tank, in which a temperature was set at 50°C, and a stirring speed was set at 50 rpm. After the reflux was completed, a distillate fraction was received in the hydrolysis tank, and after receiving 300 g of the distillate fraction, receiving was stopped. Materials in the hydrolysis tank were subjected to distillation under reduced pressure to distill off excess methanol and water (specifically, methanol was roughly distilled at the boiling point of methanol first, then water was roughly distilled at the boiling point of water, followed by further separation of distilled methanol-water mixture, and obtained methanol and water were respectively stored in a methanol storage tank and a water storage tank), where during distillation under reduced pressure, crystals continued to precipitate. After the distillation under reduced pressure was completed, a resulting solid-liquid mixture in the hydrolysis tank was filtered in a filtration unit, and a resulting filter cake was dried in a drying unit under vacuum at 65°C for 5 h to obtain 118.33 g of product (i.e., low-fluorine boron-10 enriched boric acid), with a yield of 95.73%.

### Example 3

123.6 g of the high-fluorine boron-10 enriched boric acid was weighed and charged to a reaction tank, and 1280 g of methanol was fed into the reaction tank at a molar ratio of boric acid to methanol of 1 : 20. The stirring was initiated, with a stirring speed maintained at 50 rpm, the boric acid was reacted with methanol at a reaction temperature of 55°C to form a trimethyl borate-methanol azeotrope, the trimethyl borate-methanol azeotrope was successively passed through an adsorption section of a packed rectification column (internally packed with an ion exchange resin, specifically Tulsimer^{®} CH-87 ion exchange resin, purchased from COHESION (Beijing, China) Technology Co., Ltd.. A mass of the ion exchange resin being greater than the mass of a fluoride in the high fluorine boron-10 enriched boric acid) and a rectification section (with a wire mesh packing disposed inside), and then passed through a condenser and refluxed to a reaction tank, where condensation was conducted at a temperature of -15°C, and reflux was conducted for 6 h, starting when an overhead temperature of the self-packing rectification column reached 55°C.

3090 g of ultrapure water was previously added to a hydrolysis tank, in which a temperature was set at 50°C, and a stirring speed was set at 50 rpm. After the reflux was completed, a distillate fraction was received in the hydrolysis tank, and after receiving 300 g of the distillate fraction, receiving was stopped. Materials in the hydrolysis tank were subjected to distillation under reduced pressure to distill off excess methanol and water (specifically, methanol was roughly distilled at the boiling point of methanol first, then water was roughly distilled at the boiling point of water, followed by further separation of distilled methanol-water mixture, and obtained methanol and water were respectively stored in a methanol storage tank and a water storage tank), where during distillation under reduced pressure, crystals continued to precipitate. After the distillation under reduced pressure was completed, a resulting solid-liquid mixture in the hydrolysis tank was filtered in a filtration unit, and a resulting filter cake was dried in a drying unit under vacuum at 65°C for 5 h to obtain 121.87 g of product (i.e., low-fluorine boron-10 enriched boric acid), with a yield of 98.60%.

### Comparative example 1

123.6 g of the high-fluorine boron-10 enriched boric acid was weighed and charged to a reaction tank, and 1280 g of methanol was fed into the reaction tank at a molar ratio of boric acid to methanol of 1 : 20. The stirring initiated, with a stirring speed maintained at at 50 rpm, the boric acid was reacted with methanol at a reaction temperature of 55°C to form a trimethyl borate-methanol azeotrope, the trimethyl borate-methanol azeotrope was passed through a rectification section of a packed rectification column (with a wire mesh packing disposed inside), that is, passing directly through the rectification section without an adsorption section, and then through a condenser. A condensation generation was refluxed to the reaction tank, where condensation was conducted at a temperature of -15°C, and reflux was conducted for 6 h, starting when an overhead temperature of the packed rectification column reached 55°C.

3090 g of ultrapure water was previously added to a hydrolysis tank, in which a temperature was set at 50°C, and a stirring speed was set at 50 rpm. After the reflux was completed, a distillate fraction was received in the hydrolysis tank, and after receiving 300 g of the distillate fraction, receiving was stopped. Materials in the hydrolysis tank were subjected to distillation under reduced pressure to distill off excess methanol and water (specifically, methanol was roughly distilled at the boiling point of methanol first, then water was roughly distilled at the boiling point of water, followed by further separation of distilled methanol-water mixture, and obtained methanol and water were respectively stored in a methanol storage tank and a water storage tank), where during distillation under reduced pressure, crystals continued to precipitate. After the distillation under reduced pressure was completed, a resulting solid-liquid mixture in the hydrolysis tank was filtered in a filtration apparatus, and a resulting filter cake was dried in a drying apparatus at 65°C under vacuum for 5 hours to obtain 121.31 g of product, with a yield of 98.15%.

### Comparative example 2

123.6 g of the high-fluorine boron-10 enriched boric acid was weighed and charged to a reaction tank, 1152 g of methanol was fed into the reaction tank at a molar ratio of boric acid to methanol of 1 : 18, and 10 g of a defluorination agent (specifically aluminum sulfate) was charged thereto. The stirring was initiated, with a stirring speed maintained at 50 rpm, the boric acid was reacted with methanol at a reaction temperature of 55°C to form a trimethyl borate-methanol azeotrope, the trimethyl borate-methanol azeotrope was passed through a rectification section of a packed rectification column (with a wire mesh packing disposed inside). That is, passing directly through the rectification section without an adsorption section, and then through a condenser, and refluxed to the reaction tank, where condensation was conducted at a temperature of -15°C, and reflux was conducted for 4 h, starting when an overhead temperature of the packed rectification column reached 55°C.

3090 g of ultrapure water was previously added to the hydrolysis tank, in which a temperature was set at 50°C, and a stirring speed was set at 50 rpm. After the reflux was completed, a distillate fraction was received in the hydrolysis tank, and after receiving 300 g of the distillate fraction, receiving was stopped. Materials in the hydrolysis tank were subjected to distillation under reduced pressure to distill off excess methanol and water (specifically, methanol was roughly distilled at the boiling point of methanol first, then water was roughly distilled at the boiling point of water, followed by further separation of distilled methanol-water mixture, and obtained methanol and water were respectively stored in a methanol storage tank and a water storage tank), where during distillation under reduced pressure, crystals continued to precipitate. After the distillation under reduced pressure was completed, a resulting solid-liquid mixture in the hydrolysis tank was filtered in a filtration apparatus, and a resulting filter cake was dried in a drying apparatus at 65°C under vacuum for 5 h to obtain 121.37 g of product, with a yield of 98.21%.

Table 1 shows reaction conditions as well as the product amount, product yield and fluoride ion content in product in the Examples and Comparative Examples. As can be seen from Table 1, compared with Comparative Example 1, in Examples 1 to 3 of the present disclosure, the fluoride ion content in the product can be effectively reduced by using a defluorination agent; compared with Comparative Example 2, Examples 1 to 3 of the present disclosure exhibit a better effect of defluorination by providing an adsorption section before the rectification section of the packed rectification column and packing the defluorination agent in the adsorption section. It is also known from Examples 1 to 3 that, according to the method of the present disclosure, the product yield is low when the amount of methanol used is reduced, and the reflux time is not desirably too short, otherwise, it will lead to an excessively high fluoride ion content in the product. In addition, methanol after distillation under reduced pressure using the method of the present disclosure can be recycled and reused after purification, which can reduce costs and increase benefits.

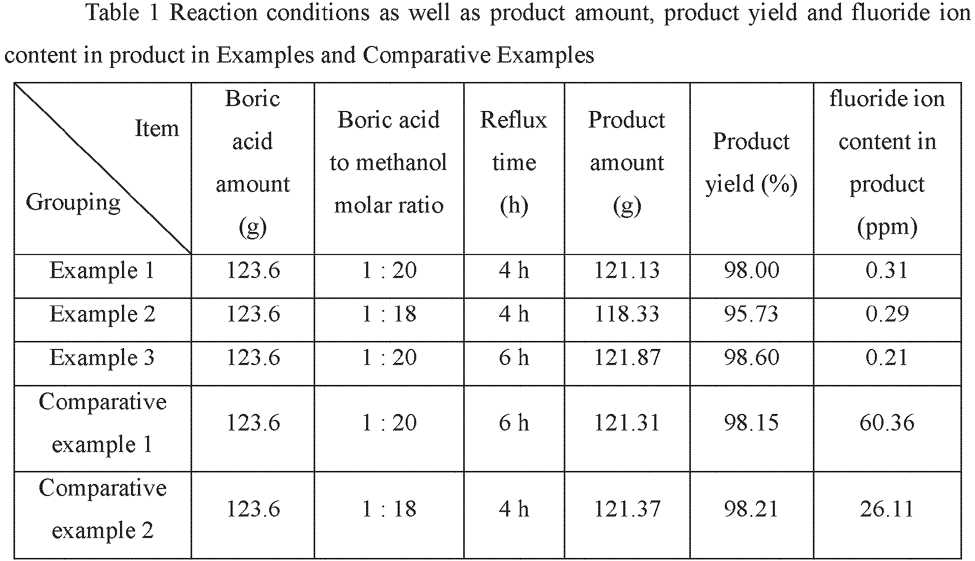

The descriptions above are merely the preferred embodiments of the present disclosure. It should be noted that several improvements and modifications may also be made by those of ordinary skill in the art without departing from the principle of the present disclosure, and these improvements and modifications should also be considered within the scope of the present disclosure.

## Claims

1. A method for preparing a low-fluorine boron-10 enriched boric acid, comprising the following steps:
subjecting a raw material liquid to a reflux treatment, subjecting a gaseous material produced during the reflux treatment to a defluorination treatment by using a defluorination agent, subjecting a resulting defluorinated material to rectification and then condensation, and returning a resulting condensed material to a cycle and conducting the reflux treatment, wherein the raw material liquid comprises a high-fluorine boron-10 enriched boric acid and methanol, the high-fluorine boron-10 enriched boric acid having a fluoride ion content of ≥ 200 ppm;
after the reflux treatment is completed, maintaining distillation and receiving a resulting distillate fraction, and mixing the resulting distillate fraction with water and subjecting a resulting mixture to a hydrolysis treatment to obtain a hydrolysate; and
concentrating the hydrolysate, and subjecting a resulting concentrated solution to solid-liquid separation to obtain the low-fluorine boron-10 enriched boric acid.

2. The method of claim 1, wherein a molar ratio of the high-fluorine boron-10 enriched boric acid to the methanol is in a range of 1 : 10 to 1 : 23.

3. The method of claim 1 or 2, wherein the reflux treatment is conducted at a heating temperature of 55°C to 60°C and a condensation temperature of -8°C to -15°C; the reflux treatment is conducted for 4 hours to 6 hours; and the reflux treatment is conducted under stirring at a speed of 50 rpm to 100 rpm.

4. The method of claim 1, wherein the defluorination agent comprises one selected from the group consisting of an ion exchange resin and aluminum sulfate.

5. The method of claim 1, wherein a mass ratio of the low-fluorine boron-10 enriched boric acid to the water is in a range of 3 : 100 to 5 : 100, based on a mass of the low-fluorine boron-10 enriched boric acid; and the water is at a temperature of 40°C to 50°C.

6. The method of claim 1 or 5, wherein the hydrolysis treatment is conducted at a temperature of 40°C to 50°C; and the hydrolysis treatment is conducted under stirring at a speed of 50 rpm to 100 rpm.

7. The method of claim 1, wherein the concentration is conducted by reduced pressure concentration; and the concentration is conducted under stirring at a speed of 50 rpm to 100 rpm.

8. The method of claim 1, wherein the method further comprises: after the solid-liquid separation, drying a wet substance obtained by the solid-liquid separation to obtain the low-fluorine boron-10 enriched boric acid.

9. An apparatus for preparing a low-fluorine boron-10 enriched boric acid, comprising a reflux unit, a hydrolysis tank, and a solid-liquid separation unit connected in sequence, wherein
the reflux unit comprises a reaction tank, a packed rectification column, and a condenser connected in sequence, wherein the packed rectification column comprises, an adsorption section and a rectification section in sequence, the adsorption section being packed with a defluorination agent and being in communication with the reaction tank; and a liquid outlet of the condenser is in communication with a reflux inlet of the rectification section and a distillate inlet of the hydrolysis tank, respectively.

10. The apparatus of claim 9, wherein the apparatus further comprises a water storage tank, a methanol storage tank, and a drying unit, wherein
a water outlet of the water storage tank is in communication with a water inlet of the hydrolysis tank, and a water inlet of the water storage tank is in communication with a water outlet of the hydrolysis tank;
a methanol inlet of the methanol storage tank is in communication with a methanol outlet of the hydrolysis tank; and
a feed inlet of the drying unit is in communication with a solid discharge outlet of the solid-liquid separation unit.
